# EUROPÄISCHE PATENTSCHRIFT

(11) **EP 3 175 802 B1**
(45) Veröffentlichungstag und Bekanntmachung des Hinweises auf die Patenterteilung: **09.06.2021**
(21) Anmeldenummer: 16002431.1
(22) Anmeldetag: 16.11.2016
(51) Int. Cl.: A61B 17/29, F16D 3/18

(54) **MEDIZINISCHES INSTRUMENT**
MEDICAL INSTRUMENT
INSTRUMENT MÉDICAL

(30) Priorität: 01.12.2015 DE 102015015655
(43) Veröffentlichungstag der Anmeldung: 07.06.2017
(73) Patentinhaber: Karl Storz SE & Co. KG, 78532 Tuttlingen (DE)
(72) Erfinder: Stefan, Jochen, 78532 Tuttlingen (DE); Kärcher, Daniel, 78532 Tuttlingen (DE)
(74) Vertreter: Hofmeister, Frank

(56) Entgegenhaltungen:
- EP-A1- 0 630 614
- EP-A1- 2 653 110
- EP-A1- 2 910 202
- DE-A1- 10 036 108
- DE-A1-102014 117 393
- DE-B3- 10 314 828
- US-A1- 2009 088 770
- US-A1- 2009 299 143

## Beschreibung

Die Erfindung betrifft ein medizinisches Instrument mit einem hohlen Schaft, an dessen proximalem Ende eine Handhabe angeordnet ist und an dessen distalem Ende ein Werkzeug mit einem starren Maulteil sowie einem relativ zu dem starren Maulteil verschwenkbaren Maulteil angeordnet ist, wobei ein das Werkzeug tragender distaler Endbereich des Schaftes als gegenüber der Längsachse des Schaftes abwinkelbare Werkzeugspitze ausgebildet ist sowie das Werkzeug um die Längsachse des Schaftes bzw. um die Längsachse der Werkzeugspitze drehbar ist, wobei das Verdrehen des Werkzeugs um die Längsachse des Schaftes über eine in dem hohlen Schaft drehbar gelagerte Betätigungsstange erfolgt, die proximalseitig mit der Handhabe in Wirkverbindung steht und wobei das Abwinkeln der Werkzeugspitze über ein axialverschiebbar im hohlen Schaft gelagertes und proximalseitig mit der Handhabe in Wirkverbindung stehendes Betätigungselement erfolgt und wobei das verschwenkbare Maulteil des Werkzeugs über ein axialverschiebbar im hohlen Schaft gelagertes und proximalseitig mit der Handhabe in Wirkverbindung stehendes Betätigungselement zwischen einer geschlossenen und eine geöffneten Position verstellbar ist.

Medizinische Instrumente für die endoskopische Chirurgie weisen in der Regel einem hohlen Schaft auf, an dessen proximalem Ende eine Handhabe und an dessen distalem Ende ein Werkzeug aus zwei relativ zueinander bewegbaren Maulteilen angeordnet ist. Das als Greif- ,Halte- und/oder Schneidinstrument ausgebildete Werkzeug ist über die Handhabe betätigbar. Um bei den häufig beengten Arbeitsverhältnissen mit dem Werkzeug einen möglichst großen Wirkungsbereich abdecken zu können, sind viele endoskopische Instrumente so ausgebildet, dass das Werkzeug gegenüber der Längsachse des Schaftes abwinkelbar ausgebildet ist sowie das Werkzeug um die Längsachse des Schaftes drehbar ist.

Ein Problem bei den aus dem Stand der Technik bekannten medizinischen Instrumenten mit diesen vielfältigen Verstellmöglichkeiten der Werkzeugspitze und/oder des distalen Werkzeugs besteht darin, dass das Abwinkeln der Werkzeugspitze relativ zum proximalen Schaftbereich eine Zwangsbewegung der Maulteile relativ zueinander und/oder eine Rotationsbewegung der Werkzeugspitze zur Folge hat. Um diese Zwangsbewegung zu beheben, sind aus der Praxis verschiedene aufwendige Ausgleichsmechanismen bekannt. Diese Ausgleichsmechanismen gewährleisten zwar eine im Wesentlichen zwangsbewegungsfreie Abwinklung der Werkzeugspitze, jedoch ist der konstruktive Aufwand sehr groß und steht der insbesondere bei endoskopischen Instrumenten erforderlichen kompakten Bauweise entgegen.

Ein gattungsgemäßes medizinisches Instrument ist beispielsweise aus der DE 103 14 828 B3 bekannt. Bei diesem bekannten chirurgischen Instrument ist ein Bewegungskompensationselement vorgesehen, das dafür sorgt, dass eine durch die Abwinklung der Werkzeugspitze verursachte Rotation kompensiert wird.

Weitere alternative Mechanismen sind aus der DE 100 36 108 A1 und der DE 10 2014 117393 A1 bekannt.

Davon ausgehend liegt der Erfindung die **Aufgabe** zugrunde, ein medizinisches Instrument der eingangs genannten Art zu schaffen, das bei kompakter Bauweise eine im Wesentlichen zwangsbewegungsfreie Abwinklung der Werkzeugspitze gewährleistet.

Die Lösung dieser Aufgabenstellung ist erfindungsgemäß durch die Merkmale von Anspruch 1 gekennzeichnet.

Durch die erfindungsgemäße Kopplung der Bewegungen des axialverschiebbaren Betätigungselements zum Abwinkeln der Werkzeugspitze mit der des axialverschiebbaren Betätigungselements zum Betätigen des verschwenkbaren Maulteils des Werkzeugs miteinander, wird die durch das Abwinkeln der Werkzeugspitze zwangsläufig bewirkte Verlagerung des starren Maulteils relativ zum verschwenkbaren Maulteil kompensiert, da nunmehr gleichzeitig das verschwenkbare Maulteil im gleichen Maße betätigt wird, was ein Beibehalten der eingestellten Position der Maulteile zueinander zur Folge hat.

Erfindungsgemäß wird vorgeschlagen, dass das axialverschiebbare Betätigungselement zum Abwinkeln der Werkzeugspitze und das axialverschiebbare Betätigungselement zum Betätigen des verschwenkbaren Maulteils des Werkzeugs in Richtung der Längsachse des Schaftes parallel zueinander angeordnet sind und den gleichen radialen Abstand zur Drehachse aufweisen, um die die Werkzeugspitze relativ zum proximalen Teil des Schaftes abwinkelbar ist. Durch den gleichen Abstand der beiden Betätigungselemente zur Drehachse wird gewährleistet, dass beide Betätigungselemente bei der gekoppelten gleichzeitigen Bewegung um die gleiche axiale Strecke verlagert werden.

Weiterhin wird mit einer bevorzugten Ausführungsform der Erfindung vorgeschlagen, dass die Betätigungsstange zum Drehen des Werkzeugs zweiteilig ausgebildet aus einem in der abwinkelbaren Werkzeugspitze gelagerten distalen Teilbereich sowie einem im proximalen Teil des Schaftes gelagerten Teilbereich besteht, wobei die beiden einander zugewandten Stirnseiten der Teilbereiche der Betätigungsstange im Übergang zur abwinkelbaren Werkzeugspitze über stirnseitige Verzahnungen miteinander in Eingriff stehen. Der zweiteilige Aufbau der Betätigungsstange zum Drehen des Werkzeugs sowie die beiden stirnseitigen Verzahnung der beiden Teilbereiche der Betätigungsstange ermöglichen eine spielarme Übertragung der Drehbewegung vom Schaftbereich auf die Werkzeugspitze.

Zur Kopplung des Werkzeugs mit dem distalen Teilbereiche der Betätigungsstange wird mit der Erfindung vorgeschlagen, dass das proximale Ende des verschwenkbaren Maulteils im Inneren des distalen Teilbereichs der um die Längsachse des Schaftes drehbaren Betätigungsstange gelagert ist und der distale Teilbereich der Betätigungsstange und das proximale Ende des verschwenkbaren Maulteils über einen die beiden Bauteile radial durchragenden Mitnahmestift kraftschlüssig so miteinander verbunden sind, dass der Mitnahmestift die Rotation des distalen Teilbereichs der Betätigungsstange direkt auf das verschwenkbare Maulteil überträgt.

Gemäß einer praktischen Ausführungsform der Erfindung wird vorgeschlagen, dass die beiderseitigen freien Enden des Mitnahmestiftes in einem Schieber gelagert sind, der von der Rotation des distalen Teilbereichs der Betätigungsstange entkoppelt axialverschiebbar in der Werkzeugspitze gelagert ist.

Zum Verstellen des verschwenkbaren Maulteils zwischen einer geschlossenen und einer offenen Position wird erfindungsgemäß vorgeschlagen, dass der Mitnahmestift so in dem Schieber gelagert ist, dass der Mitnahmestift in einer umlaufenden Nut des Schiebers frei um die Längsachse des Schaftes drehbar gelagert ist und in Richtung der Längsachse des Schaftes über den Schieber axialverschiebbar ist.

Um die Bewegung des verschwenkbaren Maulteils von der Rotation der Werkzeugspitze zu entkoppeln, wird gemäß einer bevorzugten Ausführungsform der Erfindung vorgeschlagen, dass der Mitnahmestift im distalen Teilbereich der Betätigungsstange in einem Langloch gelagert ist, dessen axiale Erstreckung dem axialen Verschiebeweg des Schiebers entspricht.

Weiterhin wird mit der Erfindung vorgeschlagen, dass das axialverschiebbare Betätigungselement zum Betätigen des verschwenkbaren Maulteils des Werkzeugs und der Schieber so miteinander gekoppelt sind, dass eine Axialverschiebung des Betätigungselements spielfrei eine Axialbewegung des Schiebers bewirkt. Die Spielfreiheit der Maulteilbetätigung ist wichtig, damit der Operateur seine Krafteinleitung genau dosieren kann und ein stets sicherer Halt zwischen den Maulteilen des Werkzeugs gewährleistet ist.

Schließlich wird mit der Erfindung vorgeschlagen, dass die Zahnflanken der einzelnen Zähne der beiden stirnseitigen Verzahnungen sich radial nach außen verjüngend ausgebildet sind. Durch diese Ausbildung der Zahnflanken der einzelnen Zähne der beiden stirnseitigen Verzahnungen wird ohne Axialausgleich ein Verkanten der beiderseitigen Zähne der stirnseitigen Verzahnungen auch bei einem Verschwenken der stirnseitigen Verzahnungen relativ zueinander ausgeschlossen.

Weitere Merkmale und Vorteile der Erfindung ergeben sich anhand der zugehörigen Zeichnungen, in denen ein Ausführungsbeispiel eines erfindungsgemäßen medizinischen Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Instruments;
- Fig. 2: einen Längsschnitt des Details II gemäß Fig. 1, eine nur teilweise abgewinkelte Position darstellend;
- Fig. 3: eine ausschnittweise Ansicht gemäß Fig. 2, jedoch den Längsschnitt in einer anderen vertikalen Ebene darstellend;
- Fig. 4: eine ausschnittweise Ansicht von unten des Details II gemäß Fig. 1;
- Fig. 5: eine Ansicht von unten auf das Detail V gemäß Fig. 2 und
- Fig. 6: eine perspektivische Ansicht einer stirnseitigen Verzahnung gemäß Fig. 2.

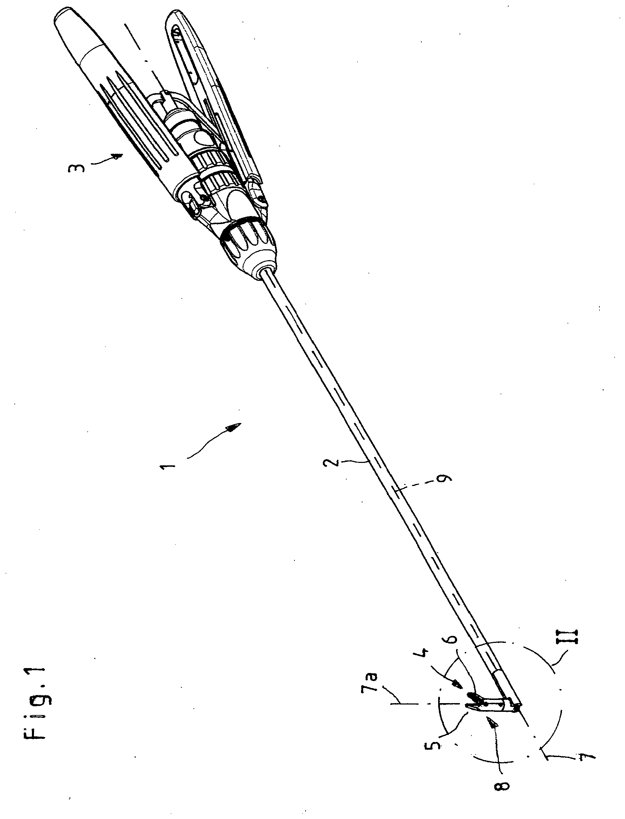

Die Abbildung Fig. 1 zeigt ein medizinisches Instrument 1 mit einem hohlen Schaft 2, an dessen proximalem Ende eine Handhabe 3 angeordnet ist und an dessen distalem Ende ein Werkzeug 4 angeordnet ist, das aus einem starren Maulteil 5 und einem gegenüber dem starren Maulteil 5 verschwenkbaren Maulteil 6 besteht.

Um dem Werkzeug 4 möglichst viele Freiheitsgrade zur Bewegung relativ zum Schaft 2 einzuräumen, ist ein das Werkzeug 4 tragender distaler Endbereich des Schaftes 2 als gegenüber der Längsachse 7 des Schaftes 2 abwinkelbare Werkzeugspitze 8 ausgebildet. In der Darstellung gemäß Fig. 1 ist die Werkzeugspitze 8 gegenüber der Längsachse 7 des Schaftes 2 etwa um 90° abgewinkelt.

Weiterhin ist das Werkzeug 4 um die Längsachse 7 des Schaftes 2 bzw. bei abgewinkelter Werkzeugspitze 8 um die Längsachse 7a der Werkzeugspitze 8 drehbar, wobei das Verdrehen des Werkzeugs 4 um die Längsachse 7 des Schaftes 2 über eine in dem hohlen Schaft 2 drehbar gelagerte Betätigungsstange 9 erfolgt, die proximalseitig mit der Handhabe 3 in Wirkverbindung steht, wobei die Betätigungsstange 9 zweiteilig ausgebildet aus einem in der abwinkelbaren Werkzeugspitze 8 gelagerten distalen Teilbereich 10 sowie einem im proximalen Teil des Schaftes 2 gelagerten Teilbereich 11 besteht.

Zur Ausbildung der Betätigungsstange 9 kann sowohl eine massive Stange als auch ein Hohlrohr verwendet werden.

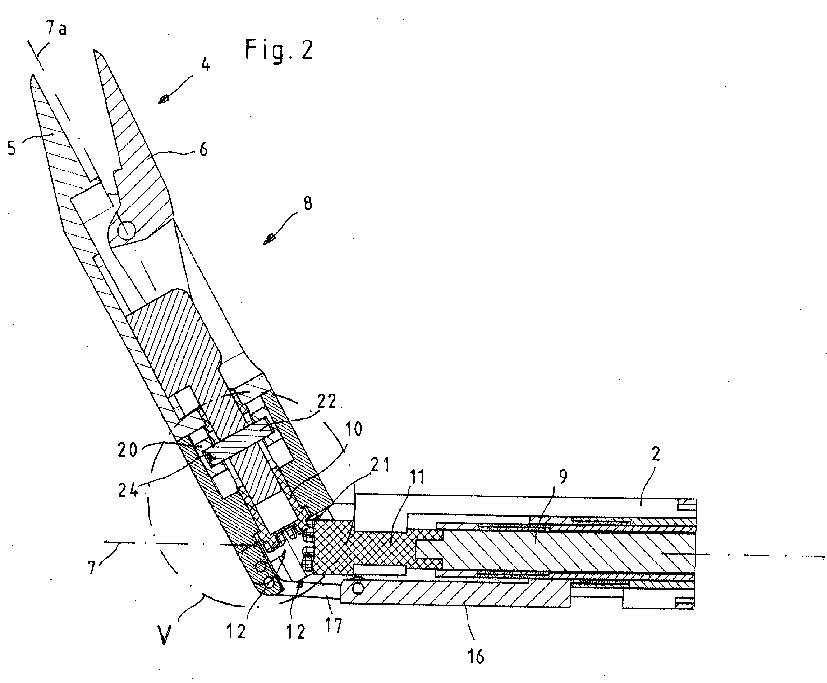

Die beiden einander zugewandten Stirnseiten der Teilbereiche 10 und 11 der Betätigungsstange 9 stehen im Übergang zur abwinkelbaren Werkzeugspitze 8 über stirnseitige Verzahnungen 12 miteinander in Eingriff, wie dies der schematischen Schnittdarstellung gemäß Fig. 2 zu entnehmen ist. Diese beiden stirnseitigen Verzahnungen 12 der beiden Teilbereiche 10 und 11 der Betätigungsstange 9 ermöglichen es, dass das Werkzeug 4 auch in einer Position, in der beide Teilbereiche 10 und 11 der Betätigungsstange 9 relativ zueinander abgewinkelt angeordnet sind, um die Längsachse 7 des Schaftes 2 drehbar ist. Die stirnseitigen Verzahnungen 12 übertragen die Rotation des proximalen Teilbereichs 11 der Betätigungsstange 9 um die Längsachse 7 des Schaftes 2 auf den distalen Teilbereich 10 der Betätigungsstange 9.

Die Abbildung Fig. 2 zeigt die Anordnung der stirnseitigen Verzahnungen 12 innerhalb der Schaftes 2 anhand eines Längsschnitts im Bereich des Übergangs vom proximalen Schaft 2 zur abgewinkelten distalen Werkzeugspitze 8.

Ein Hauptproblem bei der Ausbildung der beiden stirnseitigen Verzahnungen 12 der relativ zueinander verschwenkbaren Teilbereiche 10 und 11 der Betätigungsstange 9 besteht darin, ein gegenseitiges Verkanten und Blockieren einzelner Zähne 13 der stirnseitigen Verzahnungen 12 beim Verschwenken zu vermeiden.

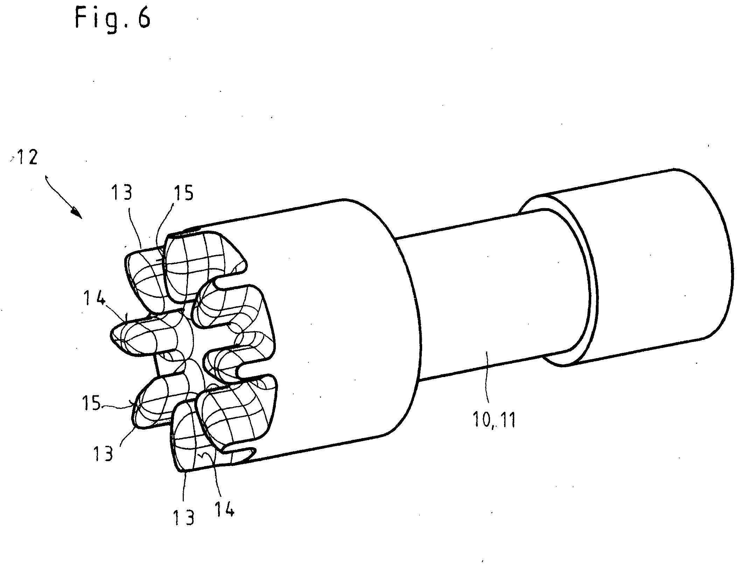

Fig. 6 zeigt eine perspektivische Ansicht einer der beiden identisch aufgebauten stirnseitigen Verzahnungen 12. Wie aus Fig. 6 ersichtlich, sind die Zahnflanken 14 der einzelnen Zähne 13 der beiden stirnseitigen Verzahnungen 12 sich radial nach außen verjüngend ausgebildet. Durch die Ausbildung der Zahnflanken 14 der einzelnen Zähne 13 der beiden stirnseitigen Verzahnungen 12 als sich, in Bezug auf die jeweilige Verzahnung 12, nach radial außen verjüngend, wird ein Verkanten der beiderseitigen Zähne 13 der stirnseitigen Verzahnungen 12 auch bei einem Verschwenken der stirnseitigen Verzahnungen 12 relativ zueinander ausgeschlossen. Diese jederzeitige Verkantungsfreiheit macht es möglich, dass auf einen Axialausgleich, beispielsweise in der Form einer Federvorspannung, verzichtet werden kann. Aufgrund der sich radial nach außen verjüngenden Ausbildung der einzelnen Zähne 13 der beiden stirnseitigen Verzahnungen 12 verbleibt gerade im radial außen liegenden Bereich für die einzelnen Zähne 13 immer ausreichend seitlicher Spielraum zu den benachbarten Zähnen 13 der jeweils anderen stirnseitigen Verzahnung 12.

Bei der in den Abbildungen dargestellten Ausführungsform der stirnseitigen Verzahnungen 12 sind zusätzlich auch die Zahnflanken 14 der einzelnen Zähne 13 der beiden stirnseitigen Verzahnungen 12 sich radial nach innen verjüngend ausgebildet, wodurch die Bewegungsfreiheit der einzelnen Zähne 13 der beiden stirnseitigen Verzahnungen 12 relativ zueinander weiter erhöht wird.

Der perspektivischen Ansicht gemäß Fig. 6 ist weiterhin zu entnehmen, dass die Zahnköpfe 15 der einzelnen Zähne 13 der beiden stirnseitigen Verzahnungen 12 abgerundet ausgebildet sind. Auch diese Abrundung der die Oberseite der einzelnen Zähne 13 bildenden Zahnköpfe 15 erleichtert weiterhin das verkantungsfreie Verschwenken der stirnseitigen Verzahnungen 12 relativ zueinander.

Neben dem verkantungsfreien Verschwenken der Werkzeugspitze 8 im Bereich der stirnseitigen Verzahnungen 12 besteht ein wesentliches Problem eines medizinischen Instruments 1 gemäß Fig. 1 darin, dass das Abwinkeln der Werkzeugspitze 8 in der Regel eine Veränderung der Lage der Maulteile 5 und 6 des Werkzeugs 4 zueinander bewirkt. In der Praxis bedeutet dies, dass sich die Maulteile 5 und 6 des Werkzeugs 4 bei der Vergrößerung der Abwinklung der Werkzeugspitze 8 zu schließen beginnen und umgekehrt bei der Verkleinerung der Abwinklung der Werkzeugspitze 8 zu öffnen beginnen. Eine derartige Zwangskopplung der Bewegungen ist zu vermeiden, um dem Operateur einen immer gleichen Zugriff des Werkzeugs 4 zu gewährleisten.

Das Abwinkeln der Werkzeugspitze 8 erfolgt, wie aus der Abbildung Fig. 2 ersichtlich, über ein axialverschiebbar im hohlen Schaft 2 gelagertes und proximalseitig mit der Handhabe 3 in Wirkverbindung stehendes Betätigungselement 16, das als Zug-/Druckstange ausgebildet ist. Mit der Werkzeugspitze 8 ist das Betätigungselement 16 über einen Anlenkhebel 17 verbunden, der proximalseitig gelenkig am Betätigungselement 16 und distalseitig gelenkig an der Werkzeugspitze 8 gelagert ist.

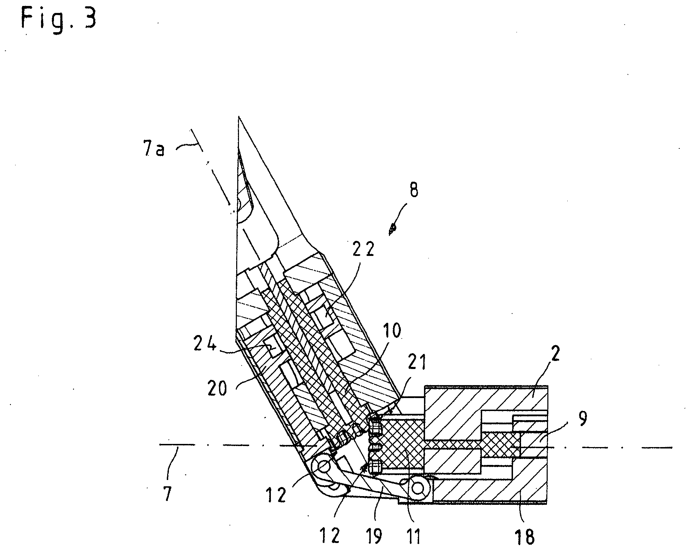

Das Verstellen des verschwenkbaren Maulteils 6 des Werkzeugs 4 zwischen einer geschlossenen und eine geöffneten Position erfolgt, wie aus der Abbildung Fig. 3 ersichtlich, über ein axialverschiebbar im hohlen Schaft 2 gelagertes und proximalseitig mit der Handhabe 3 in Wirkverbindung stehendes Betätigungselement 18, das ebenfalls als Zug-/Druckstange ausgebildet ist. Mit dem verschwenkbaren Maulteil 6 ist das Betätigungselement 18 über einen Anlenkhebel 19 verbunden, der proximalseitig gelenkig am Betätigungselement 18 und distalseitig gelenkig an einem Schieber 20 gelagert ist, der mit dem verschwenkbaren Maulteil 6 in Wirkverbindung steht.

Zur Vermeidung der unerwünschten Zwangsbewegung des Werkzeugs 4 beim Abwinkeln der Werkzeugspitze 8 sind bei dem dargestellten medizinischen Instrument 1 das axialverschiebbare Betätigungselement 16 zum Abwinkeln der Werkzeugspitze 8 und das axialverschiebbare Betätigungselement 18 zum Betätigen des verschwenkbaren Maulteils 6 des Werkzeugs 4 derart miteinander gekoppelt, dass einerseits beim Betätigen des axialverschiebbaren Betätigungselements 16 zum Abwinkeln der Werkzeugspitze 8 zwangsläufig gleichzeitig das axialverschiebbare Betätigungselement 18 zum Betätigen des verschwenkbaren Maulteils 6 des Werkzeugs 4 in Axialrichtung bewegbar ist und andererseits das axialverschiebbare Betätigungselement 18 zum Betätigen des verschwenkbaren Maulteils 6 des Werkzeugs 4 unabhängig vom axialverschiebbaren Betätigungselement 16 zum Abwinkeln der Werkzeugspitze 8 betätigbar ist.

Durch diese Kopplung der Bewegungen des axialverschiebbaren Betätigungselements 16 zum Abwinkeln der Werkzeugspitze 8 mit der des axialverschiebbaren Betätigungselements 18 zum Betätigen des verschwenkbaren Maulteils 6 des Werkzeugs 4 miteinander, wird die durch das Abwinkeln der Werkzeugspitze 8 zwangsläufig bewirkte Verlagerung des starren Maulteils 5 relativ zum verschwenkbaren Maulteil 6 kompensiert, da nunmehr gleichzeitig das verschwenbare Maulteil 6 im gleichen Maße betätigt wird, was ein Beibehalten der eingestellten Position der Maulteile 5 und 6 zueinander zur Folge hat.

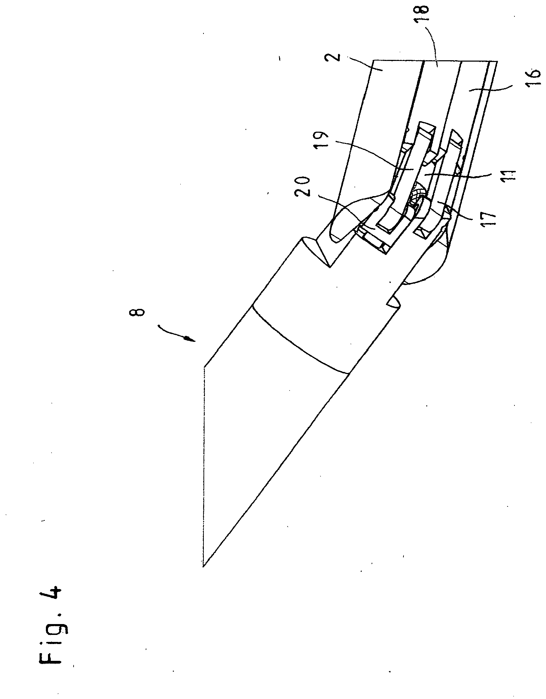

Wie aus der Zusammenschau der Untenansicht gemäß der Abbildung Fig. 4 sowie den Abbildungen Fig. 2 und 3 ersichtlich, sind das axialverschiebbare Betätigungselement 16 zum Abwinkeln der Werkzeugspitze 8 sowie der zugehörige Anlenkhebel 17 und das axialverschiebbare Betätigungselement 18 zum Betätigen des verschwenkbaren Maulteils 6 des Werkzeugs 4 sowie der zugehörige Anlenkhebel 19 in Richtung der Längsachse 7 des Schaftes 2 parallel zueinander angeordnet und weisen den gleichen radialen Abstand zur Drehachse 21 auf, um die die Werkzeugspitze 8 relativ zum proximalen Teil des Schaftes 2 abwinkelbar ist.

Aufgrund der parallelen Anordnung des axialverschiebbaren Betätigungselements 16 zum Abwinkeln der Werkzeugspitze 8 sowie des zugehörige Anlenkhebels 17 und des axialverschiebbaren Betätigungselements 18 zum Betätigen des verschwenkbaren Maulteils 6 des Werkzeugs 4 sowie des zugehörigen Anlenkhebels 19 und des gleichen radialen Abstands zur Drehachse 21 ist die Abwinklungsbewegung so abgestimmt, dass durch die Winkelbeziehung bzw. die Anordnung der Betätigungselemente 16 und 18 sowie der Anlenkhebel 17 und 19 die Maulteile 5 und 6 des Werkzeugs 4 ihre Position behalten. Die Kopplung der Betätigungselemente 16 und 18 sowie deren zuvor beschriebene konstruktiv geometrische Anordnung zueinander synchronisieren die Axialbewegungen der Betätigungselemente 16 und 18 und infolge dessen auch die Stellung der Maulteile 5 und 6 des Werkzeugs 4 zueinander.

Die Übertragung der Bewegung des axialverschiebbaren Betätigungselements 18 zum Betätigen des verschwenkbaren Maulteils 6 des Werkzeugs 4 sowie des mit dem Betätigungselement 18 gekoppelten Anlenkhebels 19 auf das verschwenkbar Maulteil 6 erfolgt über den axialverschiebbar auf dem distalen Teilbereich 10 der Betätigungsstange 9 gelagerten Schieber 20, wie sich dies aus der nachfolgend beschriebenen Zusammenschau der Abbildungen Fig. 2, 3 und 5 ergibt.

Das proximale Ende des verschwenkbaren Maulteils 6 ist im Inneren des distalen Teilbereichs 10 der um die Längsachse 7 des Schaftes 2 drehbaren Betätigungsstange 9 gelagert. Im Bereich des auf dem distalen Teilbereich 10 der Betätigungsstange 9 gelagerten Schiebers 20 durchragt ein Mitnahmestift 22 das proximale Ende des verschwenkbaren Maulteils 6 sowie den distalen Teilbereich 10 der Betätigungsstange 9 derart, dass die beiderseitigen freien Enden des Mitnahmestiftes 22 in dem Schieber 20 gelagert sind.

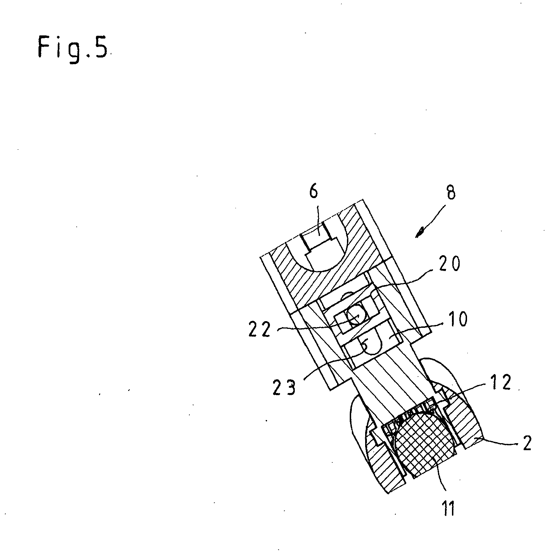

Wie aus der Ansicht gemäß der Abbildung Fig. 5 ersichtlich, ist der Mitnahmestift 22 im distalen Teilbereich 10 der Betätigungsstange 9 in einem Langloch 23 gelagert, dessen axiale Erstreckung dem axialen Verschiebeweg des Schiebers 20 entspricht.

Die Axialverschiebung des Betätigungselements 18 bewirkt somit über die gelenkige Kopplung an den Anlenkhebel 19 und den Schieber 20 eine axiale Verschiebung des Mitnahmestiftes 22 innerhalb des Langlochs 23. Aufgrund der kraftschlüssigen Kopplung des Mitnahmestiftes 22 mit dem proximalen Endes des verschwenkbaren Maulteils 6 ist so das verschwenkbare Maulteil 6 zwischen einer offenen und einer geschlossenen Position relativ zum starren Maulteil 5 verstellbar.

Der das proximale Ende des verschwenkbaren Maulteils 6 sowie den distalen Teilbereich 10 der Betätigungsstange 9 durchragende Mitnahmestift 22 verbindet die beiden Bauteile 10 und 6 kraftschlüssig so miteinander, dass der Mitnahmestift 22 die Rotation des distalen Teilbereichs 10 der Betätigungsstange 9 direkt auf das verschwenkbare Maulteil 6 überträgt, wenn der distale Teilbereich 10 der Betätigungsstange 9 unter Zwischenschaltung der stirnseitigen Verzahnungen 12 von dem im proximalen Teil des Schaftes 2 gelagerten Teilbereich 11 der Betätigungsstange 9 zu einer Rotation um die Längsachse 7 angetrieben wird.

Um den axialverschiebar in der Werkzeugspitze 8 gelagerten Schieber 20 von der Rotation des distalen Teilbereichs 10 der Betätigungsstange 9 zu entkoppeln, ist der Mitnahmestift 22 frei um die Längsachse 7 des Schaftes 2 drehbar in einer umlaufenden Nut 24 des Schiebers 20 gelagert.

Ein wie zuvor beschrieben aufgebautes medizinisches Instrument 1 zeichnet sich dadurch aus, dass eine im Wesentlichen zwangsbewegungsfreie Abwinklung der Werkzeugspitze 8 ohne Kompensationselemente möglich ist.

### Bezugszeichenliste

- 1: medizinisches Instrument
- 2: Schaft
- 3: Handhabe
- 4: Werkzeug
- 5: starres Maulteil
- 6: verschwenkbares Maulteil
- 7: Längsachse (Schaft)
- 7a: Längsachse (Werkzeugspitze)
- 8: Werkzeugspitze
- 9: Betätigungsstange
- 10: Teilbereich (distal)
- 11: Teilbereich (proximal)
- 12: stirnseitige Verzahnung
- 13: Zahn
- 14: Zahnflanke
- 15: Zahnkopf
- 16: Betätigungselement (abwinkeln)
- 17: Anlenkhebel
- 18: Betätigungselement (Maulteil)
- 19: Anlenkhebel
- 20: Schieber
- 21: Drehachse
- 22: Mitnahmestift
- 23: Langloch
- 24: Nut

## Patentansprüche

1. Medizinisches Instrument mit einem hohlen Schaft (2), an dessen proximalem Ende eine Handhabe (3) angeordnet ist und an dessen distalem Ende ein Werkzeug (4) mit einem starren Maulteil (5) sowie einem relativ zu dem starren Maulteil (5) verschwenkbaren Maulteil (6) angeordnet ist, wobei ein das Werkzeug (4) tragender distaler Endbereich des Schaftes (2) als gegenüber der Längsachse (7) des Schaftes (2) abwinkelbare Werkzeugspitze (8) ausgebildet ist sowie das Werkzeug (4) um die Längsachse (7) des Schaftes (2) bzw. um die Längsachse (7a) der Werkzeugspitze (8) drehbar ist, wobei das Verdrehen des Werkzeugs (4) um die Längsachse (7) des Schaftes (2) über eine in dem hohlen Schaft (2) drehbar gelagerte Betätigungsstange (9) erfolgt, die proximalseitig mit der Handhabe (3) in Wirkverbindung steht und wobei das Abwinkeln der Werkzeugspitze (8) über ein axialverschiebbar im hohlen Schaft (2) gelagertes und proximalseitig mit der Handhabe (3) in Wirkverbindung stehendes Betätigungselement (16) erfolgt und wobei das verschwenkbare Maulteil (6) des Werkzeugs (4) über ein axialverschiebbar im hohlen Schaft (2) gelagertes und proximalseitig mit der Handhabe (3) in Wirkverbindung stehendes Betätigungselement (18) zwischen einer geschlossenen und eine geöffneten Position verstellbar ist,
**dadurch gekennzeichnet,**
**dass** das axialverschiebbare Betätigungselement (16) zum Abwinkeln der Werkzeugspitze (8) und das axialverschiebbare Betätigungselement (18) zum Betätigen des verschwenkbaren Maulteils (6) des Werkzeugs (4) derart miteinander gekoppelt sind, dass einerseits beim Betätigen des axialverschiebbaren Betätigungselements (16) zum Abwinkeln der Werkzeugspitze (8) zwangsläufig gleichzeitig das axialverschiebbare Betätigungselement (18) zum Betätigen des verschwenkbaren Maulteils (6) des Werkzeugs (4) zur Beibehaltung der Position der Maulteile (5) und (6) zueinander in Axialrichtung bewegt wird und andererseits das axialverschiebbare Betätigungselement (18) zum Betätigen des verschwenkbaren Maulteils (6) des Werkzeugs (4) unabhängig vom axialverschiebbaren Betätigungselement (16) zum Abwinkeln der Werkzeugspitze (8) betätigbar ist, wobei das axialverschiebbare Betätigungselement (16) zum Abwinkeln der Werkzeugspitze (8) und das axialverschiebbare Betätigungselement (18) zum Betätigen des verschwenkbaren Maulteils (6) des Werkzeugs (4) in Richtung der Längsachse (7) des Schaftes (2) parallel zueinander angeordnet sind und den gleichen radialen Abstand zur Drehachse (21) aufweisen, um die die Werkzeugspitze (8) relativ zum proximalen Teil des Schaftes (2) abwinkelbar ist.

2. Medizinisches Instrument nach Anspruch 1, **dadurch gekennzeichnet, dass** die Betätigungsstange (9) zum Drehen des Werkzeugs (4) zweiteilig ausgebildet aus einem in der abwinkelbaren Werkzeugspitze (8) gelagerten distalen Teilbereich (10) sowie einem im proximalen Teil des Schaftes (2) gelagerten Teilbereich (11) besteht, wobei die beiden einander zugewandten Stirnseiten der Teilbereiche (10 und 11) der Betätigungsstange (9) im Übergang zur abwinkelbaren Werkzeugspitze (8) über stirnseitige Verzahnungen (12) miteinander in Eingriff stehen.

3. Medizinisches Instrument nach Anspruch 2, **dadurch gekennzeichnet, dass** das proximale Ende des verschwenkbaren Maulteils (6) im Inneren des distalen Teilbereichs (10) der um die Längsachse (7) des Schaftes (2) drehbaren Betätigungsstange (9) gelagert ist und der distale Teilbereich (10) der Betätigungsstange (9) und das proximale Ende des verschwenkbaren Maulteils (6) über einen die beiden Bauteile (10 und 6) radial durchragenden Mitnahmestift (22) kraftschlüssig so miteinander verbunden sind, dass der Mitnahmestift (22) die Rotation des distalen Teilbereichs (10) der Betätigungsstange (9) direkt auf das verschwenkbare Maulteil (6) überträgt.

4. Medizinisches Instrument nach Anspruch 3, **dadurch gekennzeichnet, dass** die beiderseitigen freien Enden des Mitnahmestiftes (22) in einem Schieber (20) gelagert sind, der von der Rotation des distalen Teilbereichs (10) der Betätigungsstange (9) entkoppelt axialverschiebbar in der Werkzeugspitze (8) gelagert ist.

5. Medizinisches Instrument nach Anspruch 4, **dadurch gekennzeichnet, dass** der Mitnahmestift (22) so in dem Schieber (20) gelagert ist, dass der Mitnahmestift (22) in einer umlaufenden Nut (24) des Schiebers (20) frei um die Längsachse (7) des Schaftes (2) drehbar gelagert ist und in Richtung der Längsachse (7) des Schaftes (2) über den Schieber (20) axialverschiebbar ist.

6. Medizinisches Instrument nach Anspruch 4 oder 5, **dadurch gekennzeichnet, dass** der Mitnahmestift (22) im distalen Teilbereich (10) der Betätigungsstange (9) in einem Langloch (23) gelagert ist, dessen axiale Erstreckung dem axialen Verschiebeweg des Schiebers (20) entspricht.

7. Medizinisches Instrument nach einem der Ansprüche 4 bis 6, **dadurch gekennzeichnet, dass** das axialverschiebbare Betätigungselement (18) zum Betätigen des verschwenkbaren Maulteils (6) des Werkzeugs (4) und der Schieber (20) so miteinander gekoppelt sind, dass eine Axialverschiebung des Betätigungselements (18) spielfrei eine Axialbewegung des Schiebers (20) bewirkt.

8. Medizinisches Instrument nach einem der Ansprüche 2 bis 7, **dadurch gekennzeichnet, dass** die Zahnflanken (14) der einzelnen Zähne (13) der beiden stirnseitigen Verzahnungen (12) sich radial nach außen verjüngend ausgebildet sind.

## Claims

1. Medical instrument with a hollow shank (2), at the proximal end of which a handle (3) is arranged, and at the distal end of which a tool (4) is arranged with a stationary jaw part (5) and with a jaw part (6) that is pivotable relative to the stationary jaw part (5), a distal end region of the shank (2) that carries the tool (4) being designed as a tool tip (8) that can be positioned at an angle with respect to the longitudinal axis (7) of the shank (2), and the tool (4) being rotatable about the longitudinal axis (7) of the shank (2) and/or about the longitudinal axis (7a) of the tool tip (8), the rotation of the tool (4) about the longitudinal axis (7) of the shank (2) being effected via an actuation rod (9) which is mounted rotatably in the hollow shank (2) and which is operatively connected at its proximal end to the handle (3), the tool tip (8) being positioned at an angle via an actuation element (16) which is mounted axially displaceably in the hollow shank (2) and which is operatively connected at its proximal end to the handle (3), and the pivotable jaw part (6) of the tool (4) being adjustable between a closed position and an open position via an actuation element (18) which is mounted axially displaceably in the hollow shank (2) and which is operatively connected at its proximal end to the handle (3),
**characterized in that** the axially displaceable actuation element (16) for positioning the tool tip (8) at an angle and the axially displaceable actuation element (18) for actuating the pivotable jaw part (6) of the tool (4) are coupled to each other in such a way that, on the one hand, when the axially displaceable actuation element (16) for positioning the tool tip (8) at an angle is actuated, the axially displaceable actuation element (18) for actuating the pivotable jaw part (6) of the tool (4) is necessarily movable at the same time in the axial direction so as to maintain the position of the jaw parts (5) and (6) relative to each other in the axial direction, and, on the other hand, the axially displaceable actuation element (18) for actuating the pivotable jaw part (6) of the tool (4) can be actuated independently of the axially displaceable actuation element (16) for positioning the tool tip (8) at an angle, the axially displaceable actuation element (16) for positioning the tool tip (8) at an angle and the axially displaceable actuation element (18) for actuating the pivotable jaw part (6) of the tool (4) being arranged parallel to each other in the direction of the longitudinal axis (7) of the shank (2) and being at the same radial distance from the rotation axis (21) about which the tool tip (8) can be positioned at an angle relative to the proximal part of the shank (2).

2. Medical instrument according to Claim 1, **characterized in that** the actuation rod (9) for rotating the tool (4) is composed of two parts, namely a distal sub-region (10) mounted in the pivotable tool tip (8) and a sub-region (11) mounted in the proximal part of the shank (2), and the two mutually facing end faces of the subregions (10 and 11) of the actuation rod (9) are in engagement with each other at the transition to • the pivotable tool tip (8) via end-face toothing arrangements (12).

3. Medical instrument according to Claim 2, **characterized in that** the proximal end of the pivotable jaw part (6) is mounted in the interior of the distal sub-region (10) of the actuation rod (9) rotatable about the longitudinal axis (7) of the shank (2), and the distal sub-region (10) of the actuation rod (9) and the proximal end of the pivotable jaw part (6) are connected to each other with force-fit engagement via a driving pin (22) passing radially through the two components (10 and 6) such that the driving pin (22) transmits the rotation of the distal sub-region (10) of the actuation rod (9) directly to the pivotable jaw part (6).

4. Medical instrument according to Claim 3, **characterized in that** the free ends on both sides of the driving pin (22) are mounted in a slide (20) which, decoupled from the rotation of the distal sub-region (10) of the actuation rod (9), is mounted axially displaceably in the tool tip (8) .

5. Medical instrument according to Claim 4, **characterized in that** the driving pin (22) is mounted in the slide (20) such that the driving pin (22) is mounted in a circumferential groove (24) of the slide (20) so as to rotate freely about the longitudinal axis (7) of the shank (2) and is axially displaceable in the direction of the longitudinal axis (7) of the shank (2) via the slide (20).

6. Medical instrument according to Claim 4 or 5, **characterized in that** the driving pin (22) is mounted in an oblong hole (23) in the distal sub• region (10) of the actuation rod (9), the axial extent of which oblong hole (23) corresponds to the axial displacement path of the slide (20).

7. Medical instrument according to one of Claims 4 to 6, **characterized in that** the axially displaceable actuation element (18), for actuating the pivotable jaw part (6) of the tool (4), and the slide (20) are coupled to each other such that an axial displacement of the actuation element (18) causes an axial movement of the slide (20) free from play.

8. Medical instrument according to one of Claims 2 to 7, **characterized in that** the tooth flanks (14) of the individual teeth (13) of the two end-face toothing arrangements (12) are designed tapering radially outwards.

## Revendications

1. Instrument médical comprenant une tige creuse (2), à l'extrémité proximale de laquelle une poignée (3) est disposée et à l'extrémité distale de laquelle est disposé un outil (4) pourvu d'une partie formant mâchoire immobile (5) et d'une une partie formant mâchoire (6) pouvant pivoter par rapport à la partie formant mâchoire immobile (5), une région d'extrémité distale, qui porte l'outil (4), de la tige (2) étant conçue comme une pointe d'outil (8) qui peut être inclinée par rapport à l'axe longitudinal (7) de la tige (2), et l'outil (4) pouvant tourner sur l'axe longitudinal (7) de la tige (2) ou sur l'axe longitudinal (7a) de la pointe d'outil (8), la rotation de l'outil (4) sur l'axe longitudinal (7) de la tige (2) étant effectuée par le biais d'une barre d'actionnement (9) qui est montée de manière rotative dans la tige creuse (2) et qui est reliée fonctionnellement du côté proximal à la poignée (3) et l'inclinaison de la pointe d'outil (8) étant effectuée par le biais d'un élément d'actionnement (16) qui est logé de manière mobile axialement dans la tige creuse (2) et qui est relié fonctionnellement à la poignée (3) du côté proximal, et la partie formant mâchoire pivotante (6) de l'outil (4) étant réglable entre une position fermée et une position ouverte par le biais d'un élément d'actionnement (18) qui est logé de manière déplaçable axialement dans la tige creuse (2) et qui est relié fonctionnellement à la poignée (3) du côté proximal,
**caractérisé en ce que**
l'élément d'actionnement (16) mobile axialement qui est destiné à incliner la pointe d'outil (8) et l'élément d'actionnement (18) mobile axialement qui est destiné à actionner la partie formant mâchoire pivotante (6) de l'outil (4) sont accouplés l'un à l'autre de manière que, d'une part, lorsque l'élément d'actionnement (16) mobile axialement est actionné pour incliner la pointe d'outil (8), l'élément d'actionnement (18) mobile axialement soit nécessairement déplacé en même temps dans la direction axiale pour actionner la partie formant mâchoire pivotante (6) de l'outil (4) afin de maintenir la position des parties formant mâchoire (5) et (6) l'une par rapport à l'autre et, d'autre part, l'élément d'actionnement (18) mobile axialement qui est destiné à actionner la partie formant mâchoire pivotante (6) de l'outil (4) puisse être actionné indépendamment de l'élément d'actionnement (16) mobile axialement qui est destiné à incliner la pointe d'outil (8), l'élément d'actionnement (16) mobile axialement qui est destiné à incliner la pointe d'outil (8) et l'élément d'actionnement (18) mobile axialement qui est destiné à actionner la partie formant mâchoire pivotante (6) de l'outil (4) étant disposés parallèlement l'un à l'autre dans la direction de l'axe longitudinal (7) de la tige (2) et étant à la même distance radiale de l'axe de rotation (21) sur lequel la pointe d'outil (8) peut être inclinée par rapport à la partie proximale de la tige (2).

2. Instrument médical selon la revendication 1, **caractérisé en ce que** la tige d'actionnement (9) destinée à faire tourner l'outil (4) est réalisée en deux parties et comprend une sous-zone distale (10) qui est logée dans la pointe d'outil inclinable (8) et une sous-zone distale (11) qui est logée dans la partie proximale de la tige (2), les deux faces frontales, dirigées l'une vers l'autre, des sous-zones (10 et 11) de la barre d'actionnement (9) étant en engrènement l'une avec l'autre par le biais de dentures frontales (12) dans la transition vers la pointe d'outil inclinable (8).

3. Instrument médical selon la revendication 2, **caractérisé en ce que** l'extrémité proximale de la partie formant mâchoire pivotante (6) est logée à l'intérieur de la sous-zone distale (10) de la barre d'actionnement (9) pouvant tourner sur l'axe longitudinal (7) de la tige (2) et la sous-zone distale (10) de la barre d'actionnement (9) et l'extrémité proximale de la partie formant mâchoire pivotante (6) sont reliées en force l'une à l'autre par le biais d'une broche d'entraînement (22) faisant saillie radialement à travers les deux composants (10 et 6) de sorte que la broche d'entraînement (22) transmette la rotation de la sous-zone distale (10) de la barre d'actionnement (9) directement à la partie formant mâchoire pivotante (6).

4. Instrument médical selon la revendication 3, **caractérisé en ce que** les extrémités libres des deux côtés de la broche d'entraînement (22) sont logées dans une coulisse (20) qui est logée dans la pointe de l'outil (8) de manière mobile axialement et de manière découplée de la rotation de la sous-zone distale (10) de la barre d'actionnement (9).

5. Instrument médical selon la revendication 4, **caractérisé en ce que** la broche d'entraînement (22) est logée dans la coulisse (20) de manière que la broche d'entraînement (22) soit logée dans une rainure circonférentielle (24) de la coulisse (20) de façon à pouvoir tourner librement sur l'axe longitudinal (7) de la tige (2) et soit déplaçable axialement dans la direction de l'axe longitudinal (7) de la tige (2) par le biais de la coulisse (20).

6. Instrument médical selon la revendication 4 ou 5, **caractérisé en ce que** la broche d'entraînement (22) est logée dans la sous-zone distale (10) de la barre d'actionnement (9) dans un trou allongé (23) dont l'étendue axiale correspond à la trajectoire de déplacement axial de la coulisse (20).

7. Instrument médical selon l'une des revendications 4 à 6, **caractérisé en ce que** l'élément d'actionnement (18) mobile axialement qui est destiné à actionner la partie formant mâchoire pivotante (6) de l'outil (4) et la coulisse (20) sont accouplés l'un à l'autre de manière qu'un déplacement axial de l'élément d'actionnement (18) provoque un déplacement axial de la coulisse (20) sans jeu.

8. Instrument médical selon l'une des revendications 2 à 7, **caractérisé en ce que** les flancs (14) des dents individuelles (13) des deux dentures frontales (12) sont conçus pour se rétrécir radialement vers l'extérieur.
